(19) Europäisches Patentamt
European Patent Office
Office européen des brevets

(11) Publication number: **0 008 178**
**A2**

(12) **EUROPEAN PATENT APPLICATION**

(21) Application number: **79301500.9**

(51) Int. Cl.³: **A 61 K 9/00**, A 61 K 9/14

(22) Date of filing: **27.07.79**

(30) Priority: **04.08.78 GB 3229978**
**15.03.79 GB 7909254**

(43) Date of publication of application: **20.02.80**
**Bulletin 80/4**

(84) Designated Contracting States: **BE CH DE FR GB IT NL SE**

(71) Applicant: **BEECHAM GROUP LIMITED, Beecham House Great West Road, Brentford, Middlesex (GB)**

(72) Inventor: **Clapham, David, 10 Ontario Gardens, Worthing, Sussex (GB)**
Inventor: **Treagust, Peter Glyn, 27 Greenacres, Angmering Village, Littlehampton, W. Sussex (GB)**

(74) Representative: **Dawson, Hugh Bainforde et al, Beecham Pharmaceuticals Great Burgh Yew Tree Bottom Road, Epsom Surrey KT18 5XQ (GB)**

# (54) Powder, process for its preparation, container holding it, and syrup comprising the powder reconstituted with water.

(57) A powder which may be reconstituted into an orally administrable syrup by the addition of water, which powder contains a water soluble salt of a penicillin of the formula (I):

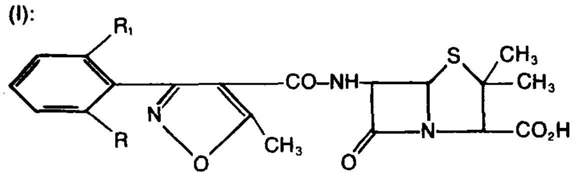

(I)

wherein R is hydrogen, chlorine or fluorine and $R_1$ is chlorine; or both R and $R_1$ are hydrogen; in mixture with a solid acidic material; the molar ratio of the penicillin to the acidic material is from 10:1 to 1:1. The powder gives a syrup of improved taste and useful stability.

EP 0 008 178 A2

TITLE MODIFIED
see front page



POWDERS

The present invention relates to powders which may be reconstituted to form orally administrable syrups.

A common method of administering medicaments is in a solution or suspension which has been reconstituted from a powder by the addition of water. For example, certain β-lactam antibiotics such as the orally administrable penicillins are often provided in admixture with excipients such as sugar, colours, preservatives, anti-foams, flavours and the like in the form of a reconstitutable powder. One disadvantage with known formulations of this kind is that they frequently allow the taste of the medicament to be detected and, in the case of an unpleasant tasting medicament, it can cause patient resistance, particularly with children, to taking the medicament.

U.K. Patent No.1511852 described a powder which may be reconstituted into an orally administrable pharmaceutical composition by the additon of water, which powder comprises (a) fine particles of a water-soluble salt of a carboxylic acid group present in a water-soluble medicament and which fine particles are

substantially or wholly coated by a water-soluble coating agent, (b) a water-soluble acidic material and (c) conventional excipients. In these powders, the taste of the medicament is rendered less apparent without seriously impairing its bioavailability.

We have now discovered improved powders for use in this way, which powders are relatively inexpensive and simple to prepare.

Accordingly, the present invention provides a powder which may be reconstituted into an orally administrable syrup by the addition of water, which powder contains a water soluble salt of a penicillin of the formula (I) :

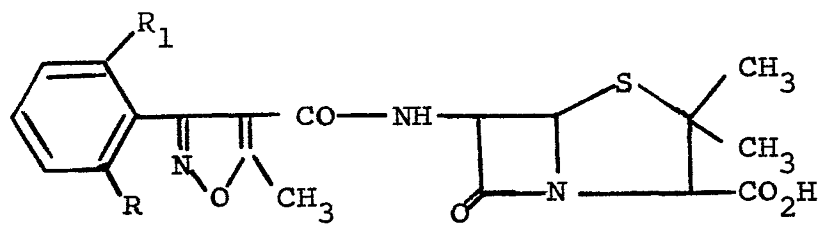

(I)

wherein R is hydrogen, chlorine, or fluorine and $R_1$ is chlorine; or both R and $R_1$ are hydrogen; in mixture with a solid acidic material; the molar ratio of the penicillin to the acidic material is from 10:1 to 1:1.

By "water soluble" is meant being capable of dissolving in water at 20°C to an extent which yields at least a 10% solution (weight/volume).

By "acidic material" is meant a pharmaceutically acceptable material which is capable of dissolving in water at 20°C to produce a pH of less than 5.5 (preferably a pH of less than 5).

In general, the powder should not contain significant numbers of penicillin particles of diameter greater than 125 microns in order to avoid

an unpalatable feeling of grittiness in the mouth, and, most suitably, at least 90% of these particles should have a diameter less than 125 microns. Preferably this diameter limit is 100 microns.

The water soluble salts of the penicillin of the formula (I) are salts of the free carboxylic acid group present in the penicillin and are usually alkali metal salts such as the sodium or potassium salts of the free carboxylic acid group.

One group of penicillins of formula (I) are those wherein R is hydrogen, chlorine or fluorine and $R_1$ is chlorine.

Suitable examples of penicillins of the formula (I) include flucloxacillin, cloxacillin, dicloxacillin and oxacillin.

A particularly suitable penicillin of the formula (I) is flucloxacillin. A preferred penicillin salt for inclusion in the powders of the invention is the sodium salt of flucloxacillin.

Suitable acidic materials for use in this invention include acids (such as organic acids), and also salts of strong acids with weak bases.

Because of their unobjectionable taste and low toxicities, particularly suitable acidic materials for use in this invention include natural non-toxic fruit acids such as citric, tartaric, malic and ascorbic acids; natural non-toxic dibasic acids such as gluconic, maleic and succinic acids; non-toxic tri-basic acids such as aconitic acid; and acid addition salts of natural non-toxic amino-acids such as glycine and glutamine. If desired mixtures of such acidic materials may be used.

Citric, tartaric, malic, maleic and aconitic acids and glycine hydrochloride are examples of particularly suitable acidic materials for use in the powders of this invention.

Preferred acidic materials include tartaric and maleic acids, and glycine hydrochloride.

The acidic material should be present in the powder in particles of size in the range 50-500 microns, though due to the solubility of the acidic material this is not an important consideration. Examples of suitable sized are as for the penicillin particles as hereinbefore described.

The ratio of penicillin to acidic material present expressed on a molar basis is from 10:1 to 1:1, more suitably 7:1 to 2:1, for example 4:1, 3:1 or 2:1.

We have found that particularly good results are obtained when this ratio is 4:1 with tartaric acid, 2:1 with maleic acid or 3:1 with glycine hydrochloride.

From the aforesaid it may be seen that a preferred embodiment of the invention is a powder comprising sodium flucloxacillin in mixture with a solid acidic material; the molar ratio of the penicillin to the acidic material is from 7:1 to 2:1.

Suitable and preferred examples of acidic materials and molar ratios in this embodiment are as hereinbefore described.

The powders of this invention will normally of course contain conventional excipients found in conventional reconstitutable powders. For example, the powders may contain sugar, flavours, artificial sweeteners, preservatives, surfactants, anti-foams, and the like in conventional quantities. Such excipients will be well known to those skilled in formulating penicillins or cephalosporins, and will of course be chosen so as to be compatible with the other powder ingredients both in the dry form and on reconstitution.

The powders of the present invention may be presented in multidose form for example in bottles or similar containers, or in single dose form for example in sachets or the like. When reconstituted the powders of the invention have acceptable stability.

Normally the powders of this invention will be in multi-dose bottles such that when they are reconstituted with water the resultant suspension will contain 62.5 mg., 125 mg., or 250 mg., of free penicillin per 5 ml of suspension.

Suitably the constitution of the powders of this invention will be such that the pH value of the suspension resulting from the admixture of the powders with water is in the range 4.0 to 5.5

The powders of this invention may be prepared by mixing methods such as those conventionally used in the preparation of reconstitutable pharmaceutical penicillin or cephalosporin powders.

Suitable methods of mixing include mixing in a 'Y-cone blender'. (Y-cone is a Registered Trade Mark).

The particles of penicillin and acidic material may be prepared by methods of comminution conventionally used in the preparation of pharmaceutical particles, such as by fine milling.

The penicillin and acidic material may be comminuted either together or separately. Where the acidic material is a mixture of different acidic materials, the components thereof may also be comminuted either together or separately.

The invention also provides a method of treatment of bacterial infections, which method comprises administering to the sufferer an effective amount of a reconstituted powder of this invention.

The 'effective amount' will be the quantity of powder necessary to give an effective dose of the penicillin included therein.

If desired the powders of this invention may additionally contain a further penicillin, for example ampicillin trihydrate or amoxycillin trihydrate.

In an alternative aspect, this invention provides a method of improving the taste of syrups reconstituted from powders containing a water soluble salt of a penicillin of formula (I), which method comprises including in the syrup a solid acidic material in the hereinbefore defined molar ratio.

The following Examples illustrate the invention.

EXAMPLE 1

A reconstitutable powder was prepared from :

| | | |
|---|---|---|
| Sodium flucloxacillin | | 125 mg |
| Tartaric Acid | | 10 mg |
| Flavours | | 25 mg |
| Sodium chloride | | 2.5 mg |
| Trisodium citrate | | 11 mg |
| Thickener | | 10 mg |
| Sugar | to | 3.75 g |

Sodium flucloxacillin and tartaric acid were milled together and blended with the remaining components.

EXAMPLE 2

As Example 1 but omitting the sodium chloride.

EXAMPLES 3 to 6

The process described in Example 1 was repeated, substituting tartaric acid by the acidic material shown below.

| Example | Acidic Material | Weight |
|---|---|---|
| 3 | Aconitic acid | 4 mg |
| 4 | Malic acid | 10 mg |
| 5 | Maleic acid | 15 mg |
| 6 | Glycine hydrochloride | 10 mg |

EXAMPLE 7

The powders prepared according to each of Examples 1-6 were made up to 5 ml with water to form orally administrable pleasant tasting syrups.

EXAMPLE 8

The syrup prepared from the powder of Example 1 was tested for stability and was found to be of acceptable stability for at least seven days at 15°C.

EXAMPLE 9

A reconstitutable powder was prepared from:

| | | | |
|---|---|---|---|
| Sodium flucloxacillin | | 125 | mg |
| Tartaric Acid | | 7 | mg |
| Flavours | | 25 | mg |
| Trisodium citrate | | 11 | mg |
| Sodium saccharin | | 2.5 | mg |
| Thickener | | 10 | mg |
| Sugar | to | 3.75 | g |

The sodium flucloxacillin and tartaric acid were milled together and blended with the remaining components.

EXAMPLE 10

Example 9 was repeated but no flavours were used.

EXAMPLE 11

A reconstituted powder was prepared from:

| | | | |
|---|---|---|---|
| Sodium flucloxacillin | | 250 | mg |
| Tartaric acid | | 25 | mg |
| Flavours | | 50 | mg |
| Trisodium citrate | | 22 | mg |
| Sodium saccharin | | 5.0 | mg |
| Thickener | | 10 | mg |
| Sugar | to | 3.75 | g |

The sodium flucloxacillin and tartaric acid were milled together and blended with the remaining components.

EXAMPLE 12

Example 11 was repeated but no flavours were used.

EXAMPLE 13

The powders prepared according to each of the Examples 9 and 11 were made up to 5 ml. with water to form orally administrable pleasant tasting syrups.

EXAMPLE 14

The powders prepared according to each of the Examples 1 to 6 and 9 to 12 were filled into separate unit dose sachets.

EXAMPLE 15

The syrup prepared in Example 13 from the powder of Example 11 had acceptable stability for seven days at 15°C.

EXAMPLE 16

Examples 9 to 14 were repeated exactly, but using sodium cloxacillin in place of the sodium fluxlcxacillin.

EXAMPLE 17

Examples 9 to 14 were repeated exactly, but using

sodium dicloxacillin in place of the sodium flucloxacillin.

What we claim is:

1. A powder which may be reconstituted into an orally administrable syrup by the addition of water, which powder contains a water soluble salt of a pencillin of the formula (I):

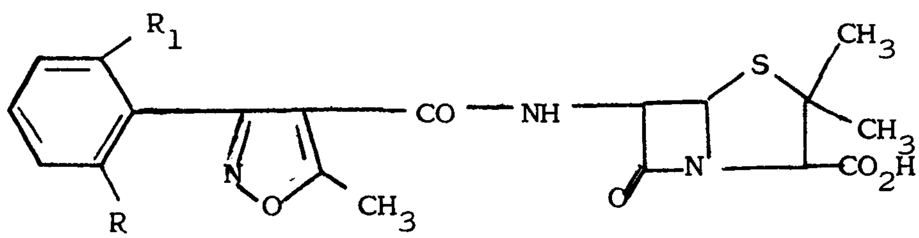

(I)

wherein R is hydrogen, chlorine or fluorine and $R_1$ is chlorine; or both R and $R_1$ are hydrogen; in mixture with a solid acidic material; the molar ratio of the penicillin to the acidic material is from 10:1 to 1:1.

2. A powder according to claim 1 wherein the pencillin salt is sodium flucloxacillin.

3. A powder according to claim 1 or claim 2 wherein the acidic material is citric, tartaric, malic, maleic or aconitric acid, or glycine hydrochloride.

4. A powder according to any one of the claims 1 to 3, wherein the acidic material is a mixture of acids.

5. A powder according to any one of the claims 1 to 4, wherein the said molar ratio is 7:1 to 2:1.

6. A container holding a single or multi-dose of the powder according to any one of the preceding claims.

7. A syrup comprising a powder according to any one of the claims 1 to 5 reconstituted with water.

8. A process for the preparation of a powder according to any one of the claims 1 to 5, which process comprises mixing together the powder ingredients.